# EUROPEAN PATENT APPLICATION

(11) **EP 3 170 826 A1**
(43) Date of publication of application: **24.05.2017**
(21) Application number: 16202511.8
(22) Date of filing: 13.12.2012
(51) Int. Cl.: C07D 487/04, A61K 31/519, A61P 35/00

(54) **TOFACITINIB MONO-MALATE SALT**

(30) Priority: 15.12.2011 US 201161576199 P
(62) Divisional of application: 12805898.9
(71) Applicant: ratiopharm GmbH, 89079 Ulm (DE)
(72) Inventor: COUTABLE, Ludovic, 89077 Ulm (DE); ALBRECHT, Wolfgang, 89075 Ulm (DE); GUSERLE, Richard, 89359 Kötz (DE)
(74) Representative: D Young & Co LLP

(57) **Abstract**

The present invention is directed to Tofacitinib mono-malate salt. The Tofacitinib mono-malate salt can be in amorphous form. The invention is also directed towards a pharmaceutical composition comprising Tofacitinib mono-malate and a process for preparing the composition. The Tofacitinib mono-malate salt can be used to prepare Tofacitinib mono-citrate salt. Another aspect of the invention is a process for preparing Tofacitinib mono-citrate. The Tofacitinib mono-malate salt is also useful as a medicament for the treatment of cancer.

## Description

### Field of the Invention

The present invention encompasses Tofacitinib salts, solid state forms thereof and pharmaceutical compositions thereof.

### Background of the Invention

Tofacitinib, 3-{(3R,4R)-4-methyl-3-[methyl-(7H-pyrrolo[2,3-d]pyrimidin-4-yl)-amino]-piperidin-1-yl}3-oxo-propionitrile, having the following formula: 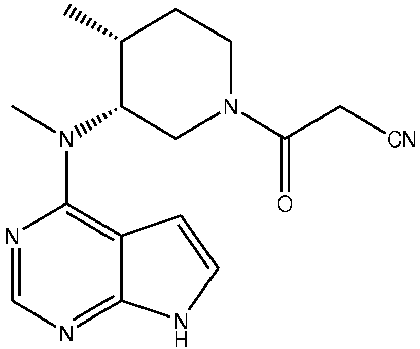 is under investigation as an inhibitor of protein kinases, such as the enzyme Janus Kinase 3 ("JAK3"). It has also been investigated as an immunosuppressive agent for the therapy of several conditions such as organ transplants, xeno transplantation, lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia and other indications, where immunosuppression would be desirable (see WO 03/048126).

Tofacitinib, as well as certain pharmaceutically acceptable salts thereof, is described in WO 01/042246 and WO 02/096909. WO 03/048162 describes crystalline and amorphous forms of the mono-citrate salt of Tofacitinib. WO 12/135338 describes processes for preparing certain other Tofacitinib salts.

The present invention relates to salts of Tofacitinib, in particular, Tofacitinib mono-tartrate, Tofacitinib mono-malate and Tofacitinib mono-oxalate, as well as to solid state forms thereof. Solid state properties of Tofacitinib salts can be influenced by controlling the conditions under which the salts, e.g., Tofacitinib mono-tartrate, Tofacitinib mono-malate and Tofacitinib mono-oxalate, are obtained in solid form.

Polymorphism, the occurrence of different crystal forms, is a property of some molecules and molecular complexes. A single molecule may give rise to a variety of polymorphs having distinct crystal structures and physical properties like melting point, thermal behaviors (e.g. measured by thermogravimetric analysis - "TGA", or differential scanning calorimetry - "DSC"), X-ray diffraction pattern, infrared absorption fingerprint, and solid state NMR spectrum. One or more of these techniques may be used to distinguish different polymorphic forms of a compound.

Different salts and solid state forms of an active pharmaceutical ingredient may possess different properties. Such variations in the properties of different salts and solid state forms may provide a basis for improving formulation, for example, by facilitating better processing or handling characteristics, improving the dissolution profile, or improving stability and shelf-life. These variations in the properties of different salts and solid state forms may also provide improvements to the final dosage form, for instance, if they serve to improve bioavailability. Different salts and solid state forms of an active pharmaceutical ingredient may also give rise to a variety of polymorphs or crystalline forms, which may in turn provide additional opportunities to assess variations in the properties and characteristics of a solid active pharmaceutical ingredient.

Discovering new salts and solid state forms of a pharmaceutical product can provide materials having desirable processing properties, such as ease of handling, ease of processing, storage stability, ease of purification or as desirable intermediate crystal forms that facilitate conversion to other polymorphic forms. New salts and solid state forms of a pharmaceutically useful compound can also provide an opportunity to improve the performance characteristics of a pharmaceutical product. It enlarges the repertoire of materials that a formulation scientist has available for formulation optimization, for example by providing a product with different properties, e.g., better processing or handling characteristics, or improved shelf-life. For at least these reasons, there is a need for additional salts and solid state forms of Tofacitinib, in particular there is a need for salts and solid state forms that have improved solubility.

### Summary of the Invention

The present invention provides salts of Tofacitinib, particularly Tofacitinib mono-tartrate, Tofacitinib mono-malate and Tofacitinib mono-oxalate, solid state forms thereof, and pharmaceutical compositions containing them.

The present invention also encompasses the use of any one of the Tofacitinib salts and solid state forms of the present invention for the preparation of other Tofacitinib salts, in particular the mono-citrate salt, solid state forms and/or pharmaceutical compositions thereof.

The present invention also provides a process for preparing Tofacitinib salts, in particular a mono-citrate salt, by preparing any one of the Tofacitinib salts and solid state forms disclosed herein, and converting it to another Tofacitinib salt, preferably, the mono-citrate.

The present invention also encompasses the Tofacitinib salts and solid state forms described herein for use as medicaments, particularly for the treatment of cancer.

The present invention further provides a pharmaceutical composition comprising any one of the Tofacitinib salts, and solid state forms thereof of the present invention, and at least one pharmaceutically acceptable excipient, for use as medicaments, particularly for the treatment of cancer. Processes for preparing the above pharmaceutical compositions are also provided.

The present invention also provides a method of treating a cancer, particularly a cancer mediated by a protein kinase, such as the enzyme Janus Kinase 3 ("JAK3"), the method comprising administering a therapeutically effective amount of at least one of the Tofacitinib salts and solid state forms thereof of the present invention, or at least one of the above pharmaceutical compositions to a person suffering from cancer or in need of the treatment.

### Brief Description of the Drawings

Figure 1 shows an ¹H-NMR spectrum of Tofacitinib mono-tartrate.
Figure 2 shows an Fourier Transform Infrared (FTIR) spectrum of Tofacitinib mono-tartrate.
Figure 3 shows an X-ray powder diffractogram of Tofacitinib mono-tartrate.
Figure 4 shows an ¹H-NMR spectrum of Tofacitinib mono-malate.
Figure 5 shows an FTIR spectrum of Tofacitinib mono-malate.
Figure 6 shows an X-ray powder diffractogram of Tofacitinib mono-malate.
Figure 7 shows an ¹H-NMR spectrum of Tofacitinib mono-oxalate.
Figure 8 shows an FTIR spectrum of Tofacitinib mono-oxalate.
Figure 9 shows an X-ray powder diffractogram of Tofacitinib mono-oxalate.

### Detailed Description of the Invention

The present invention encompasses the following new salts and solid state forms of Tofacitinib: Tofacitinib mono-tartrate, Tofacitinib mono-malate and Tofacitinib mono-oxalate, and solid state forms thereof.

In some embodiments, salts of Tofacitinib according to the invention are substantially free of any other salts of Tofacitinib, and solid state forms according to the invention are substantially free of any other polymorphic forms, or of specified polymorphic forms of Tofacitinib. In any embodiment of the present invention, by "substantially free" is meant that the salts and solid state forms of the present invention contain 20% (w/w) or less, 10% (w/w) or less, 5% (w/w) or less, 2% (w/w) or less, particularly 1% (w/w) or less, more particularly 0.5% (w/w) or less, and most particularly 0.2% (w/w) or less of any other salts and polymorphs, respectively, or of a specified polymorph of Tofacitinib. In other embodiments, the salts, e.g., Tofacitinib mono-tartrate, Tofacitinib mono-malate and Tofacitinib mono-oxalate and solid state forms thereof according to the invention can contain from 1% to 20% (w/w), from 5% to 20% (w/w), or from 5% to 10% (w/w) of any other salts, or solid state forms, or of a specified polymorph of Tofacitinib.

The new Tofacitinib salts of the present invention (particularly, mono-tartrate, mono-malate and mono-oxalate salts) have advantageous properties selected from at least one of: chemical purity, flowability, solubility, dissolution rate, morphology or crystal habit, stability- such as thermal and mechanical stability to polymorphic conversion, stability to dehydration and/or storage stability, low content of residual solvent, a lower degree of hygroscopicity, flowability, and advantageous processing and handling characteristics such as compressibility, and bulk density. In particular, the Tofacitinib salts of the present invention (particularly, mono-tartrate, mono-malate and mono-oxalate salts) have higher solubility in comparison to Tofacitinib mono-citrate. This property expands the methods of formulating that can be used. Furthermore, the amorphous forms of the Tofacitinib salts of the present invention (particularly, mono-tartrate, mono-malate and mono-oxalate salts) are polymorphically stable, i.e. do not convert to other crystalline forms.

A crystal form may be referred to herein as being characterized by graphical data "as depicted in" a Figure. Such data include, for example, powder X-ray diffractograms and solid state NMR spectra. The skilled person will understand that such graphical representations of data may be subject to small variations, e.g., in peak relative intensities and peak positions due to factors such as variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of a Tofacitinib salt referred to herein as being characterized by graphical data "as depicted in" a Figure will thus be understood to include any crystal forms of the Tofacitinib salt characterized with the graphical data having such small variations, as are well known to the skilled person, in comparison with the Figure.

As used herein, the term "isolated" in reference to any of Tofacitinib salts or solid state forms thereof of the present invention corresponds to Tofacitinib salt or solid state form thereof that is physically separated from the reaction mixture, where it is formed.

As used herein, unless stated otherwise, the XRPD measurements are taken using copper Kα radiation wavelength 1.5406 Å.

A thing, *e.g.,* a reaction mixture, may be characterized herein as being at, or allowed to come to "room temperature, often abbreviated "RT." This means that the temperature of the thing is close to, or the same as, that of the space, *e.g.,* the room or fume hood, in which the thing is located. Typically, room temperature is from about 20°C to about 30°C, or about 22°C to about 27°C, or about 25°C.

A process or step may be referred to herein as being carried out "overnight." This refers to a time interval, *e.g*., for the process or step, that spans the time during the night, when that process or step may not be actively observed. This time interval is from about 8 to about 20 hours, or about 10-18 hours, typically about 16 hours.

As used herein, the term "reduced pressure" refers to a pressure of about 10 mbar to about 50 mbar.

As used herein, the terms "vol." or "volume" can be used to refer to ml per gram of the corresponding Tofacitinib. For example, a statement that 0.5 g of Tofacitinib is dissolved in ten volumes of a Solvent X would be understood to mean that the 0.5 g of Tofacitinib was dissolved in 5 ml of Solvent X.

The present invention comprises novel salts of Tofacitinib, in particular: tartrate, malate and oxalate salts. The above salts can be isolated.

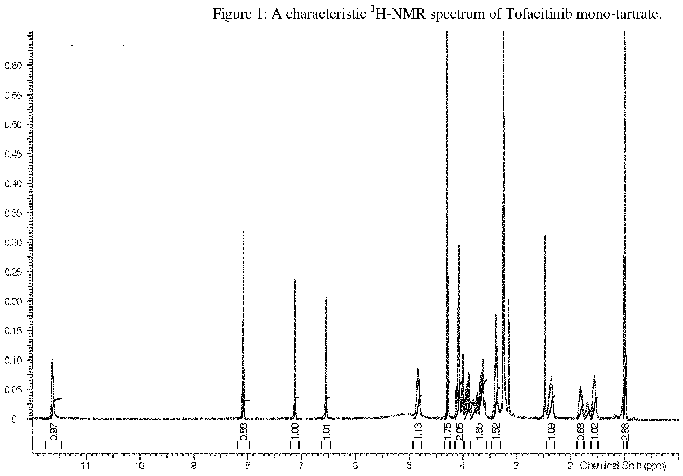

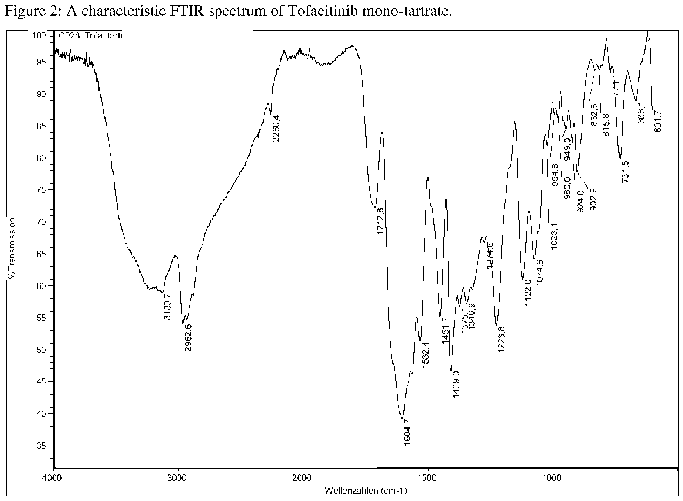

In one embodiment, the present invention comprises Tofacitinib mono-tartrate salt. The Tofacitinib mono-tartrate salt can be characterized by data selected from: an ¹H-NMR spectrum substantially as depicted in Figure 1, an FTIR spectrum with peaks at: 2963, 1713, 1605, 1532, 1452, 1409, 1227, 1122, 1075, 903, 732 cm⁻¹; an FTIR spectrum substantially as depicted in Figure 2; and combinations thereof.

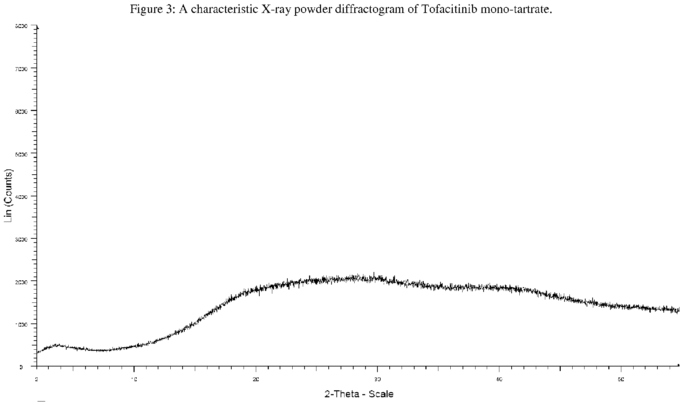

The Tofacitinib mono-tartrate may be in amorphous form. The amorphous form of Tofacitinib mono-tartrate salt can be characterized by an X-ray powder diffraction pattern substantially as depicted in Figure 3.

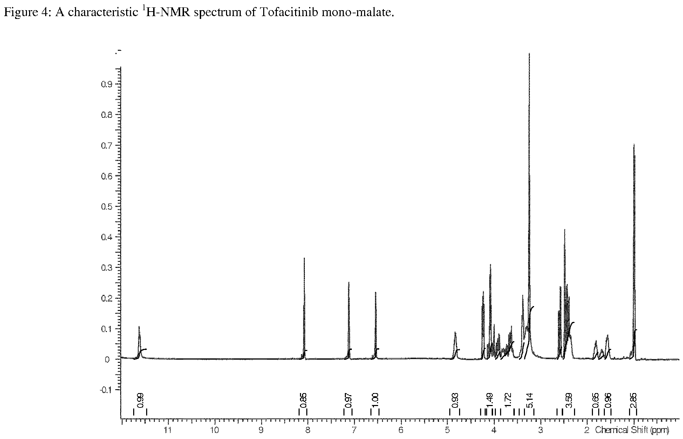

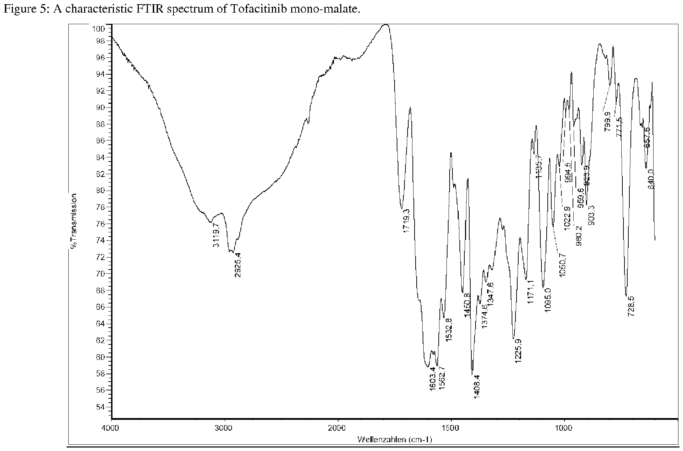

In another embodiment, the present invention comprises Tofacitinib mono-malate salt. The Tofacitinib mono-malate salt can be characterized by data selected from: an ¹H-NMR spectrum substantially as depicted in Figure 4, an FTIR spectrum with peaks at: 2925, 1719, 1603, 1563, 1533, 1451, 1408, 1226, 1095, 903, 729 cm⁻¹, an FTIR spectrum substantially as depicted in Figure 5; and combinations thereof.

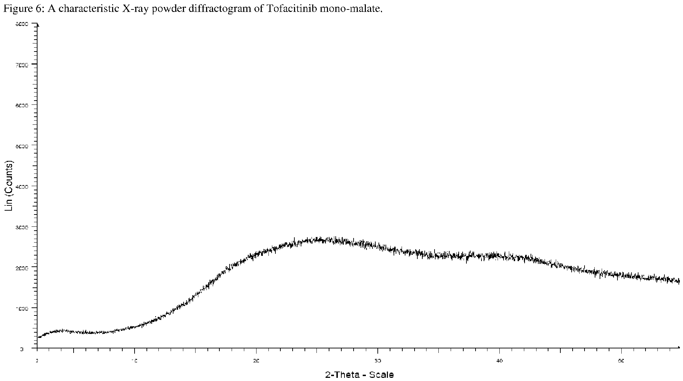

The Tofacitinib mono-malate may be in amorphous form. The amorphous form of Tofacitinib mono-malate salt can be characterized by X-ray powder diffraction pattern substantially as depicted in Figure 6.

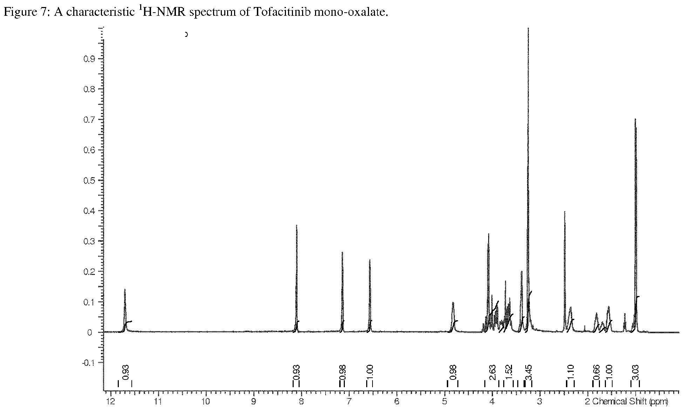

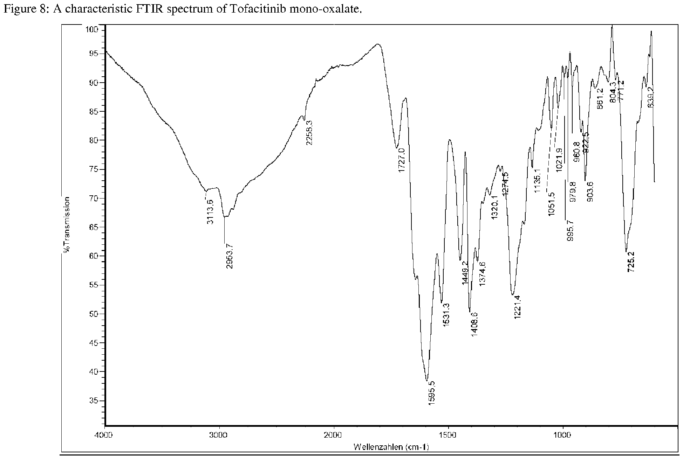

In yet another embodiment, the present invention comprises Tofacitinib mono-oxalate salt. The Tofacitinib mono-oxalate salt can be characterized by data selected from: an ¹H-NMR spectrum substantially as depicted in Figure 7, an FTIR spectrum with peaks at: 2954, 1727, 1596, 1531, 1449, 1409, 1221, 904, 725 cm⁻¹, an FTIR spectrum substantially as depicted in Figure 8; and combinations thereof.

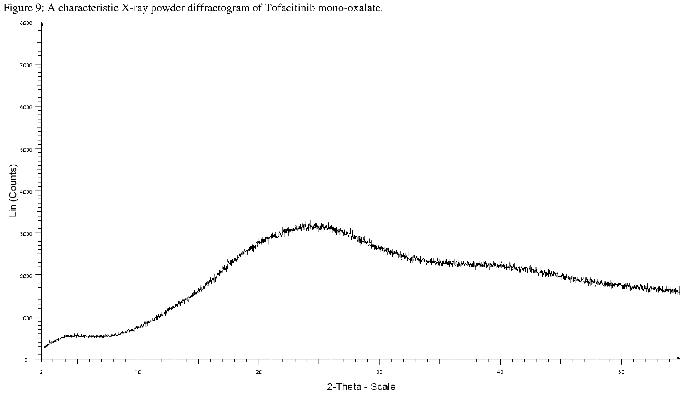

The Tofacitinib mono-oxalate may be in amorphous form. The amorphous form of Tofacitinib mono-oxalate salt can be characterized by X-ray powder diffraction pattern substantially as depicted in Figure 9.

The Tofacitinib salts of the present invention may be prepared by a process comprising: dissolving Tofacitinib in a suitable solvent to form a solution; and adding the corresponding acid to the solution.

The above described salts and solid state forms of Tofacitinib can be used to prepare other salts of Tofacitinib, in particular the mono-citrate salt, as well as solid state forms and/or pharmaceutical formulation thereof.

The present invention encompasses a process for preparing Tofacitinib salts, in particular a mono-citrate salt, the process comprising preparing any one of the Tofacitinib salts and solid state forms of Tofacitinib, particularly, Tofacitinib mono-tartrate, Tofacitinib mono-malate and Tofacitinib mono-oxalate, by the processes of the present invention, and converting that salt to said other Tofacitinib salt, preferably, Tofacitinib mono-citrate. The conversion can be done, for example, by a process comprising basifying a solution of any one or more of the above described Tofacitinib salts and/or solid state forms thereof to produce Tofacitinib base, and reacting the obtained Tofacitinib base with a suitable acid, to obtain the corresponding salt. Suitable acids include inorganic acids, such as hydrobromic acid, hydrochloric acid, sulfuric acid or phosphoric acid; and organic acids, such as oxalic acid, maleic acid, malonic acid, D-/L- or D/L-tartaric acid, fumaric acid, citric acid, D-/L- or D/L-malic acid, succinic acid, D-/L- or D/L-mandelic acid, D-/L- or D/L-lactic acid, acetic acid, propionic acid, paratoluenesulfonic acid, ethane-1,2-disulfonic acid, ethanesulfonic acid, methanesulfonic acid, naphthalene-2-sulfonic acid or benzenesulfonic acid

The Tofacitinib salts and/or solid state forms thereof of the present invention can also be used as a medicament.

The present invention further encompasses 1) a pharmaceutical composition comprising any one or more of the Tofacitinib salts and solid state forms thereof, as described above, and at least one pharmaceutically acceptable excipient; and 2) the use of any one or more of the above-described Tofacitinib salts and solid state forms thereof, in the manufacture of a pharmaceutical composition, and 3) a method of treating a person suffering from cancer, comprising administration of an effective amount of a pharmaceutical composition comprising any one or more of the forms of Tofacitinib salts described herein.

The above pharmaceutical compositions can be prepared by a process comprising combining Tofacitinib salt and solid state forms thereof (particularly, Tofacitinib mono-tartrate, Tofacitinib mono-malate and Tofacitinib mono-oxalate) with at least one pharmaceutically acceptable excipient.

Having thus described the invention with reference to particular preferred embodiments and illustrative examples, those in the art can appreciate modifications to the invention as described and illustrated that do not depart from the spirit and scope of the invention as disclosed in the specification. The Examples are set forth to aid in understanding the invention but are not intended to, and should not be construed to limit its scope in any way.

### Nuclear magnetic resonance (NMR) spectroscopy

Instrument: Varian Mercury 400 Plus NMR Spectrometer, Oxford AS, 400 MHz

### Infrared Spectroscopy

| | |
|---|---|
| Instrument: | Thermo Nicolet, Avatar 330 FT-IR. Smart Endurance Diamond-ATR |
| Software : | Omnic Vers. 6.1a |

### X-Ray Powder Diffraction

The sample was analyzed on a D8 Advance X-ray powder diffractometer (Bruker-AXS, Karlsruhe, Germany). The sample holder was rotated in a plane parallel to its surface at 20 rpm during the measurement. Further conditions for the measurements are summarized in the table below. The raw data were analyzed with the program EVA (Bruker-AXS, Germany).

| | standard measurement |
|---|---|
| radiation | Cu K_{α} (λ = 1.5406 Å) |
| source | 38kV/40mA |
| detector | Vantec |
| detector slit | variable |
| divergence slit | v6 |
| antiscattering slit | v6 |
| 2θ range / ° | 2≤2θ≤55 |
| step size / ° | 0.017 |

### Examples

### Reference examples: Preparation of Tofacitinib

Tofacitinib citrate (24.3 g; 48.2 mmol) and dichloromethane (DCM) (200 ml) were mixed and stirred in a 21 Erlenmeyer flask. A 10 % (w/v) aqueous potassium carbonate solution (305 ml) was added, and the mixture was stirred for an additional 5 min at RT and then transferred into a separation funnel. The organic phase was separated and the aqueous phase was extracted with an additional 200 ml DCM. The combined organic extract was washed with saturated sodium hydrogen carbonate (100 ml), then dried over sodium sulphate, followed by filtration and evaporation of the solvent. The residue was then dissolved in 150 ml methanol and concentrated. The resulting residue was dried at 50°C/100 mbar for 4 h, followed by 65°C/20 mbar O/N to produce Tofacitinib base (15.0 g, 99.2 %). The starting Tofacitinib citrate can be prepared for example according to the process described in "Mild and Efficient DBU-Catalyzed Amidation of Cyanoacetates" Org. Lett., 2009, Vol. 11, No. 9.

### Example 1: Preparation of Tofacitinib mono-tartrate salt

Tofacitinib (As produced in the reference example) (0.25 g) was dissolved in 2.5 ml of ethanol. L-Tartaric acid (0.18 g) was added and the resulting suspension was heated to reflux until a clear solution was obtained, stirred for 5 min and allowed to cool down slowly to room temperaure with stirring. A precipitate formed and was isolated by filtration, and dried under reduced pressure to yield 0.32 g of Tofacitinib mono-tartrate as a white powder.

### Example 2: Preparation of Tofacitinib mono-malate salt

Tofacitinib (0.10 g) (As produced in the reference example) was dissolved in 1.0 ml of ethanol. D,L-Malic acid (0.043 g) was added and the resulting suspension was heated to reflux, until a clear solution was obtained. Then, the solution was allowed to cool down to room temperature and stirred for 3 h at room temperature. Diethylether (2 mL) was added in one portion and the resulting precipitate was collected by filtration. The collected precipitate was dissolved in 2 ml of methanol and the solution was concentrated. The thus-obtained solid residue was dried under vacuum to yield 0.13 g of Tofacitinib mono-malate as a white powder.

### Example 3: Preparation of Tofacitinib mono-oxalate salt

Tofacitinib (0.10 g) (As produced in the reference example)was dissolved in 1.0 ml of ethanol. Oxalic acid dihydrate (0.040 g) was added in portions. This suspension was then heated to reflux, until a clear solution was obtained, and then allowed to cool down to room temperature and stirred for 3 h at room temperature. The volatiles were distilled off and the crude product was dissolved in 2 ml of isopropanol and concentrated. The thus-obtained solid residue was dried under vacuum to yield 0.13 g of Tofacitinib mono-oxalate as a white powder.

### Example 4: Solubility of Tofacitinib salts

The solubility of Tofacitinib salts was investigated in 0.1 N HCl (pH 1.2), 20 mM sodium acetate (pH 4.5) and 50 mM potassium dihydrogen phosphate (pH 6.8). For each salt, 250 mg of the salt was weighed into a test tube and 2.5 ml solvent was added. After mixing, the suspension was stirred for 1 h at room temperature. A sample was withdrawn, filtered and analyzed by HPLC with UV-detection (λ=290 nm). Quantification of Tofacitinib was based on a calibration curive, which was established prior to the solubility test. For Tofacitinib tartrate, Tofacitinib malate and Tofacitinib oxalate, clear solutions were obtained, indicating that saturation was not yet achieved. Therefore, for these salts, a new experiment was performed by mixing 250 mg solid substance with 2.5 ml solvent. The results are summarized in the table below.

| **compound** | **solid state** | **batch no.** | **solubility [mg/ml] pH 1.2** | **pH 4.5** | **pH 6.8** |
|---|---|---|---|---|---|
| TOF base | amorphous | LC229 | 36,7 | 6,9 | 2,1 |
| TOF-citrate | crystalline | LC125_Pr2 | 29,4 | 9,4 | 11,6 |
| TOF-citrate | amorphous | LC190* | 29,7 | 3,7 | 4,1 |
| TOF-citrate | amorphous | LC259** | N/D | N/D | 5,4 |
| TOF-tartrate | amorphous | LC242 | 381 | 324 | 353 |
| TOF-malate | amorphous | LC243 | 361 | 364 | 377 |
| TOT-oxalate | amorphous | LC247 | 375 | 329 | 350 |

| | | | | | |
|---|---|---|---|---|---|
| *: LC190 prepared by milling of Tofacitinib base with citric acid (400 rpm, 60 min) **: LC259 prepared by milling of Tofacitinib base with citric acid (400 rpm, 2x60 min) | | | | | |

Further aspects and embodiments of the invention are set out in the numbered clauses below:
1. Amorphous Tofacitinib mono-tartrate salt.
2. The amorphous Tofacitinib mono-tartrate salt according to clause 1, characterized by X-ray powder diffraction pattern substantially as depicted in Figure 3.
3. Tofacitinib mono-malate salt.
4. The Tofacitinib mono-malate salt according to clause 3, characterized by data selected from: an ¹H-NMR spectrum substantially as depicted in Figure 4, an FTIR spectrum with peaks at: 2925, 1719, 1603, 1563, 1533, 1451, 1408, 1226, 1095, 903, and 729 cm⁻¹, an FTIR spectrum substantially as depicted in Figure 5; and combinations thereof.
5. The Tofacitinib mono-malate salt according to any one of clauses 3 or 4 in amorphous form.
6. The amorphous form of Tofacitinib mono-malate salt according to clause 5, characterized by an X-ray powder diffraction pattern substantially as depicted in Figure 6.
7. Tofacitinib mono-oxalate salt.
8. The Tofacitinib mono-oxalate salt according to clause 7, characterized by data selected from: an ¹H-NMR spectrum substantially as depicted in Figure 7, an FTIR spectrum with peaks at: 2954, 1727, 1596, 1531, 1449, 1409, 1221, 904, and 725 cm⁻¹, an FTIR spectrum substantially as depicted in Figure 8; and combinations thereof.
9. The Tofacitinib mono-oxalate salt according to any one of clauses 7 or 8 in amorphous form.
10. The amorphous form of Tofacitinib mono-oxalate salt according to clause 9, characterized by an X-ray powder diffraction pattern substantially as depicted in Figure 9.
11. A pharmaceutical composition comprising one or more of the Tofacitinib salts and solid state forms thereof according to any one of clauses 1-10 and at least one pharmaceutically acceptable excipient.
12. A process for preparing the pharmaceutical composition according to clause 11 comprising combining one or more of the Tofacitinib salts and solid state forms thereof according to any one of clauses 1-10 with at least one pharmaceutically acceptable excipient.
13. Use of the Tofacitinib salts and solid state forms according to any one of clauses 1-10 to prepare Tofacitinib mono-citrate salt.
14. A process for preparing a Tofacitinib mono-citrate salt comprising preparing any one of the Tofacitinib salts and solid state forms thereof according to any one of clauses 1-10 and converting it to Tofacitinib mono-citrate.
15. The process according to clause 14, wherein the converting is done by a process comprising basifying a solution of one or more of the Tofacitinib salts and/or solid state forms thereof according to any one of clauses 1-10 to produce Tofacitinib base, and reacting the Tofacitinib base with citric acid, to obtain the Tofacitinib mono-citrate.
16. Use of the Tofacitinib salts and/or solid state forms according to any one of clauses 1-10 as a medicament.
17. Use of one or more of the Tofacitinib salts and solid state forms thereof according to any one of clauses 1-10, in the manufacture of a pharmaceutical composition.
18. A method of treating a person suffering from cancer, comprising administering an effective amount of a pharmaceutical composition according to clause 11.

## Claims

1. Tofacitinib mono-malate salt.

2. The Tofacitinib mono-malate salt according to claim 1 in amorphous form.

3. A pharmaceutical composition comprising Tofacitinib mono-malate salt according to any one of claims 1-2 and at least one pharmaceutically acceptable excipient.

4. A process for preparing the pharmaceutical composition according to claim 3 comprising combining Tofacitinib mono-malate salt according to any one of claims 1-2 with at least one pharmaceutically acceptable excipient.

5. Use of Tofacitinib mono-malate salt according to any one of claims 1-2 to prepare Tofacitinib mono-citrate salt.

6. A process for preparing a Tofacitinib mono-citrate salt comprising preparing Tofacitinib mono-malate salt according to any one of claims 1-2 and converting it to Tofacitinib mono-citrate.

7. The process according to claim 6, wherein the converting is done by a process comprising basifying a solution of Tofacitinib mono-malate salt according to any one of claims 1-2 to produce Tofacitinib base, and reacting the Tofacitinib base with citric acid, to obtain the Tofacitinib mono-citrate.

8. Tofacitinib mono-malate salt according to any one of claims 1-2 for use as a medicament.

9. Use of Tofacitinib mono-malate salt according to any one of claims 1-2, in the manufacture of a pharmaceutical composition.

10. Tofacitinib mono-malate salt according to any one of claims 1-2, or a pharmaceutical composition according to claim 3 for use in the treatment of cancer.
